# EUROPEAN PATENT APPLICATION

(11) **EP 1 325 928 A1**
(43) Date of publication of application: **09.07.2003**
(21) Application number: 01961343.9
(22) Date of filing: 04.09.2001
(51) Int. Cl.: C07J 5/00

(54) **PROCESS FOR THE PREPARATION OF PREGNANE DERIVATIVES**

(30) Priority: 08.09.2000 JP 2000273387
(71) Applicant: Kuraray Co., Ltd., Okayama 710-8622 (JP)
(72) Inventor: NAKAZAWA, Makoto, c/o KURARAY CO., LTD., Kurashiki-shi, Okayama 710-0801 (JP); KITA, Satoru, Kurashiki-shi, Okayama 710-0801 (JP); OHZONO, Shigeo, Kurashiki-shi, Okayama 710-0801 (JP)
(74) Representative: VOSSIUS & PARTNER
(86) International application number: JP0107639
(87) International publication number: WO02020552

(57) **Abstract**

A process for preparing pregnane derivatives represented by the formula (V), 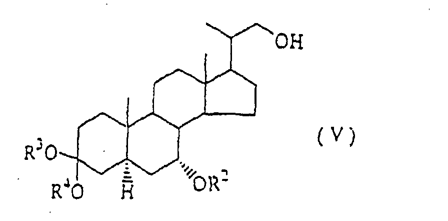 which comprises the steps of:
reacting an alkali (earth) metal with a compound represented by the formula (I) 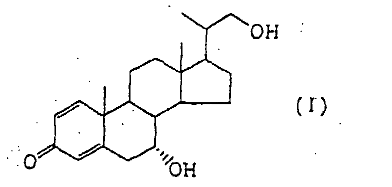
in the presence of ammonia or an amine, to obtain a compound represented by the formula (II); 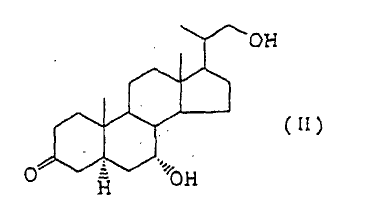
protecting the hydroxy groups of the obtained compound, to obtain a compound represented by the formula (III); 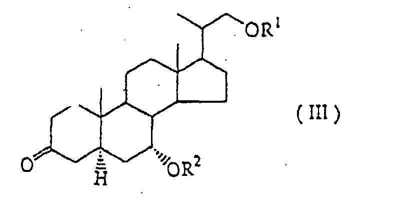
protecting the 3-position carbonyl group of the obtained compound, to obtain a compound represented by the formula (IV); 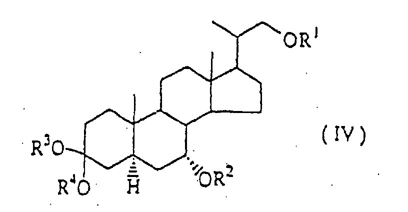
and subjecting the obtained compound to solvolysis; and the compound (II).

## Description

### TECHNICAL FIELD

The present invention relates to processes for preparing pregnane derivatives. The pregnane derivatives obtained by the processes of the present invention are useful, for example, as intermediates for synthesis of squalamine represented by the following formula.

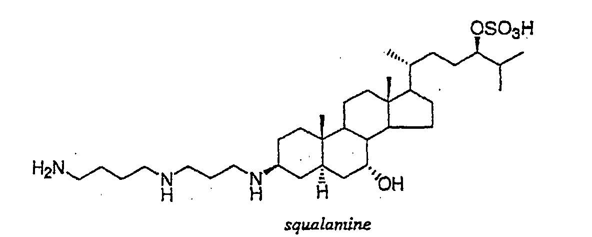

Squalamine has been reported to exhibit potent antimicrobial activity against Gram-positive bacteria, Gram-negative bacteria and fungi, as well as an anti-carcinogenic activity and has been attracting much attention as a new antibiotic [see, for example, J. Org. Chem. 63, 3786(1998); J. Org. Chem. 63, 8599(1998); and WO 98/24800].

### BACKGROUND ART

Squalamine has been extracted from the livers of dogfish sharks at such a low extraction efficiency of 0.001 to 0.002% by weight that its chemical synthesis has been investigated. Known processes of chemical synthesis of squalamine include 1) a process which comprises using 3 β -acetoxy-5-cholenic acid as a starting material [see Tetrahedron Lett., 35, 8103(1994)]; 2) a process which comprises using 3 β -hydroxy-5-cholenic acid as a starting material [see J. Org. Chem. 60, 5121(1995) and WO 94/19366]; 3) a process which comprises using 21-hydroxy-20-methyl-pregna-4-en-3-one as a starting material [see WO 98/24800]; and 4) a process which comprises using stigmasterol as a starting material [see J. Org. Chem. 63, 3786(1998), J. Org. Chem. 63, 8599(1998), and WO 98/24800].

The 3 β -acetoxy-5-cholenic acid, 3 β -hydroxy-5-cholenic acid and 21-hydroxy-20-methyl-pregna-4-en-3-one used in the above processes 1), 2) and 3), respectively, are all expensive. Moreover, the above processes 1), 2) and 3) include complex reaction operations, requiring 17 steps, 19 steps and 15 steps, respectively, to obtain squalamine. Accordingly, these processes can hardly be said to be suitable for commercial production.

The above process 4), using a starting material of stigmasterol available at a low cost, requires 20 steps to synthesize squalamine. Moreover, the process has the drawbacks of using an expensive silver carbonate in a step of selectively oxidizing the 3-position hydroxy group and, further, of comprising an ozone-oxidation step under a low temperature, which requires a special reaction equipment. This process cannot necessarily be said to be commercially advantageous.

Accordingly, an object of the present invention is to provide a process for preparing pregnane derivatives from a readily available starting material and efficiently in short steps.

### DISCLOSURE OF THE INVENTION

In order to achieve the above object, the present inventors have made an intensive study, while selecting as a starting material 7 α -hydroxy-3-oxo-pregna-1,4-dime-20-carbaldehyde which is readily obtainable by microbiologically transforming 3 α ,7 α -dihydroxy-5 β -cholenic acid and/or derivatives thereof (see Japanese Patent No. 2525049).

Synthesis of 7 α -hydroxy-3-oxo-5 α -cholic acid by reacting metallic lithium with 7 α -hydroxy-3-oxo-1,4-choladienic acid in the presence of ammonia is known [see Chemical and Pharmaceutical Bulletin 41, 763(1993)]. The present inventors applied this reaction to 7 α ,21-dihydroxy-20-methyl-pregna-1,4-dien-3-one (see Reference Example 1) which is obtainable by reduction of the 20-position aldehyde of 7 α -hydroxy-3-oxo-pregna-1,4-diene-20-carbaldehyde. As a result, the present inventors found that 7 α ,21-dihydroxy-20-methyl-5 α -pregna-3-one was obtained at a good yield. This 7 α ,21-dihydroxy-20-methyl-5 α -pregna-3-one is a novel compound, although 7 α ,21-dihydroxy-20-methyl-5 β-pregna-3-one is known as an intermediate for synthesis of chenodeoxycholic acid from 3-keto-bisnorcholenol (see EP 0 018 515 A2).

Thus, the present invention provides:
(1) a process for preparing 21-hydroxypregnane derivatives represented by the general formula (V) (hereinafter referred to as "21-hydroxypregnane derivatives (V)") 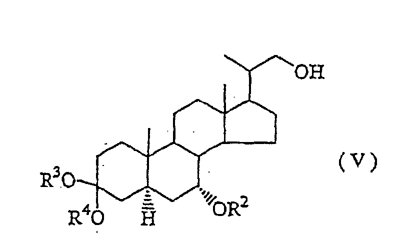 wherein R² represents a protecting group for hydroxy group, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted, which comprises the steps of:
   reacting an alkali metal or an alkali earth metal with a compound represented by the formula (I), i.e. 7 α ,21-dihydroxy-20-methyl-pregna-1,4-dien-3-one (hereinafter referred to as "compound (I)"), 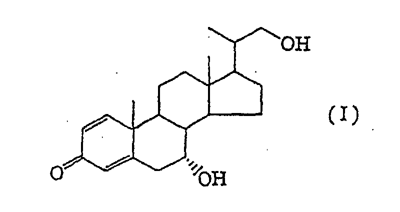
   in the presence of ammonia or an amine, to obtain a compound represented by the formula (II), i.e. 7 α ,21-dihydroxy-20-methyl-5 α -pregna-3-one (hereinafter referred to as "compound (II)"); 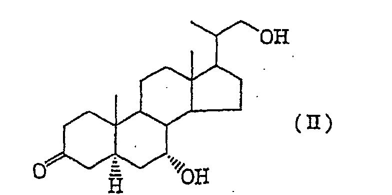
   protecting the hydroxy groups of the obtained compound (II), to obtain a 3-oxopregnane derivative represented by the general formula (III) (hereinafter referred to as "3-oxopregnane derivative (III)") 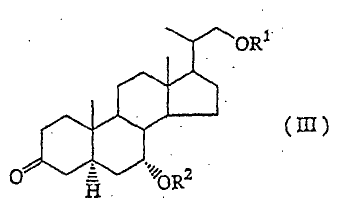
   wherein R¹ and R² each represents a protecting group for hydroxy group;
   protecting the 3-position carbonyl group of the obtained 3-oxop-regnane derivative (III), to obtain a pregnane derivative represented by the general formula (IV) (hereinafter referred to as "pregnane derivative (IV)") 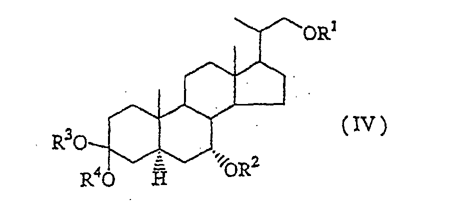 wherein R¹ and R² are as defined above and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted; and
   subjecting the obtained pregnane derivative (IV) to solvolysis;
(2) the process according to the above (1), which comprises the steps of:
   reacting an alkali metal or an alkali earth metal with a compound represented by the formula (I-1), i.e. (20S)-7 α ,21-dihydroxy-20-methyl-pregna-1,4-dien-3-one (hereinafter referred to as
   "compound (I-1)"), 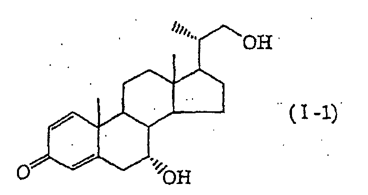
   in the presence of ammonia or an amine, to obtain a compound represented by the formula (II-1), i.e. (20S)-7 α ,21-dihydroxy-20-methyl-5 α -pregna-3-one (hereinafter referred to as "compound (II-1)"); 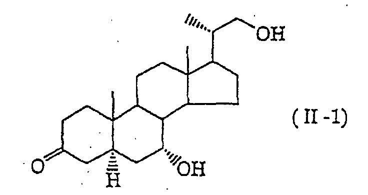
   protecting the hydroxy groups of the obtained compound (II-1), to obtain a 3-oxopregnane derivative represented by the general formula (III-1) (hereinafter referred to as "3-oxopregnane derivative (III-1)") 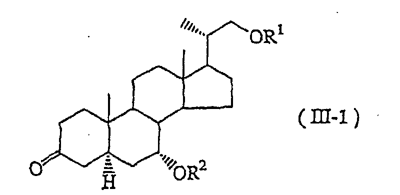
   wherein R¹ and R² each represents a protecting group for hydroxy group;
   protecting the 3-position carbonyl group of the obtained 3-oxopregnane derivative (III-1), to obtain a pregnane derivative represented by the general formula (IV-1) (hereinafter referred to as "pregnane derivative (IV-1)") 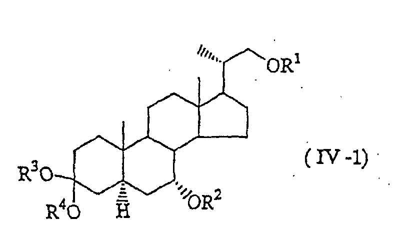 wherein R¹ and R² are as defined above and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted; and
   subjecting the obtained pregnane derivative (IV-1) to solvolysis, to obtain a 21-hydroxypregnane derivative represented by the general formula (V-1) (hereinafter referred to as "21-hydroxypregnane derivative (V-1)") 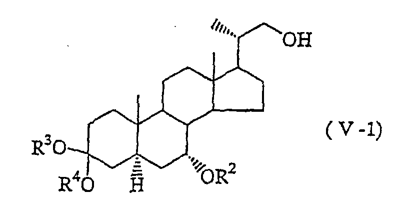 wherein R¹, R², R³ and R⁴ are as defined above;
(3) a process for preparing the compounds (II), which comprises reacting an alkali metal or an alkali earth metal with compound (I) in the presence of ammonia or an amine;
(4) the process according to the above (3), which comprises reacting an alkali metal or an alkali earth metal with compound (I-1) in the presence of ammonia or an amine, to obtain compound (II-1);
(5) a process for preparing pregnane derivatives (IV), which comprises protecting the hydroxy groups of compound (II), to obtain 3-oxopregnane derivative (III), and protecting the 3-position carbonyl group of the obtained 3-oxopregnane derivative (III);
(6) the process according to the above (5), which comprises protecting the hydroxy groups of compound (II-1), to obtain 3-oxopregnane derivative (III-1), and protecting the 3-position carbonyl group of the obtained 3-oxopregnane derivative (III-1), to obtain pregnane derivative (IV-1);
(7) a process for preparing 21-hydroxypregnane derivatives (V), which comprises subjecting pregnane derivative (IV) to solvolysis;
(8) the process according to the above (7), which comprises subjecting pregnane derivative (IV-1) to solvolysis, to obtain 21-hydroxypregnane derivative (V-1);
(9) compound (II), i.e. 7 α ,21-dihydroxy-20-methyl-5 α -pregna-3-one;
(10) compound (II-1), i.e. (20S)-7 α ,21-dihydroxy-20-methyl-5 α-pregna-3-one, which is an embodiment of the compound (II) of the above (9);
(11) a process for preparing 21-hydroxypregnane derivatives (V), which comprises subjecting 3-oxopregnane derivative (III) to solvolysis, to obtain a 21-hydroxy-3-oxopregnane derivative represented by the general formula (VI) (hereinafter referred to as "21-hydroxy-3-oxopregnane derivative (VI)") 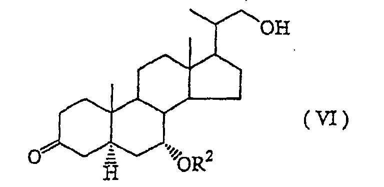 wherein R² is as defined above,
   and protecting the 3-position carbonyl group of the obtained 21-hydroxy-3-oxopregnane derivative (VI); and
(12) the process according to the above (11), which comprises subjecting 3-oxopregnane derivative (III-1) to solvolysis, to obtain a 21-hydroxy-3-oxopregnane derivative represented by the general formula (VI-1) (hereinafter referred to as "21-hydroxy-3-oxopregnane derivative (VI-1)") 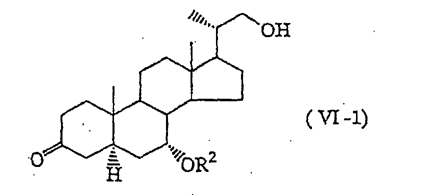
wherein R² is as defined above,
and protecting the 3-position carbonyl group of the obtained 21-hydroxy-3-oxopregnane derivative (VI-1), to obtain 21-hydroxypregnane derivative (V-1).

### BEST MODES FOR CARRYING OUT THE INVENTION

Any protecting group can be used as the protecting group for hydroxy group represented by each of R¹ and R², as long as it can act as such. Examples of the protecting group are alkyl groups, e.g. tert-butyl and tert-amyl; aralkyl groups, e.g. benzyl, o-methylbenzyl, m-methylbenzyl, p-methylbenzyl, p-nitrobenzyl, p-methoxybenzyl, p-phenylbenzyl, diphenylmethyl and triphenylmethyl; acyl groups; e.g. acetyl, chloroacetyl, dichloroacetyl, trichloroacetyl, trifluoroacetyl, methoxyacetyl, triphenylmethoxyacetyl, phenoxyacetyl, p-chlorophenoxyacetyl, phenylacetyl, diphenylacetyl, propionyl, butyryl, valeryl, 4-pentenoyl, pivaloyl, crotonoyl, benzoyl, o-methylbenzoyl, m-methylbenzoyl, p-methylbenzoyl, 2,3-dimethylbenzoyl, 2,4-dimethylbenzoyl, 2,5-dimethylbenzoyl, 2,6-dimethylbenzoyl, 2,4,6-trimethylbenzoyl, and p-phenylbenzoyl; alkoxycarbonyl groups, e.g. methoxycarbonyl, 9-fluorenylmethoxycarbonyl, ethoxycarbonyl, 2,2,2-trichloroethoxycarbonyl, 1,1-dimethyl-2,2,2-trichloroethoxycarbonyl and 2-(trimethylsilyl)ethoxycarbonyl; alkenyloxycarbonyl groups, e.g. vinyloxycarbonyl and allyloxycarbonyl; aryloxycarbonyl groups, e.g. phenoxycarbonyl and p-nitrophenoxycarbonyl; aralkyloxycarbonyl groups, e.g. benzyloxycarbonyl, p-methoxybenzyloxycarbonyl, 3,4-dimethoxybenzyloxycarbonyl, o-nitrobenzyloxycarbonyl, p-nitrobenzyloxycarbonyl, 2-(4-nitrophenyl)ethoxycarbonyl and 2-(2,4-dinitrophenyl)ethoxycarbonyl; alkoxyalkyl groups, e.g. methoxymethyl, benzyloxymethyl, p-methoxybenzyloxymethyl, p-nitrobenzylmethoxymethyl, o-nitrobenzylmethoxymethyl, (4-methoxyphenoxy)methyl, text-butoxymethyl, 2-methoxyethoxymethyl and 2-(trimethylsilyl)ethoxymethyl; oxacycloalkyl groups, e.g. tetrahydro-2-furanyl and tetrahydro-2-pyranyl; and trisubstituted silyl groups, e.g. trimethylsilyl, ethyldimethylsilyl, isopropyldimethylsilyl, tert-butyldimethylsilyl, triethylsilyl, tert-butyldiphenylsilyl and triphenylsilyl.

Those alkyl groups that have 1 to 6 carbon atoms are preferably used as the alkyl group which may be represented by each of R³ and R⁴. Examples of such alkyl groups are methyl, ethyl, n-propyl, isopropyl, n-butyl, 1-methylpropyl, 2-methylpropyl, n-pentyl, 1-methylbutyl, 1,1-dimethylpropyl, 1,2-dimethylpropyl, 2,2-dimethylpropyl, 1-ethylpropyl and n-hexyl. These alkyl groups may be substituted. Examples of the subatituting groups for this purpose are hydroxy group; halogen atoms, e.g. fluorine, chlorine, bromine and iodine; alkoxy groups, e.g. methoxy, ethoxy, propoxy and butoxy; and aralkyloxy groups, e.g. benzyloxy.

Those alkenyl groups that have 3 to 6 carbon atoms are preferably used as the the alkenyl group which may be represented by each of R³ and R⁴. Examples of such alkenyl groups are 2-propenyl, 2-butenyl, 3-butenyl, 1-methyl-2-propenyl, 2-methyl-2-propenyl, 2-pentenyl, 3-pentenyl, 4-pentenyl, 1-methyl-2-butenyl, 1-methyl-3-butenyl, 2-methyl-2-butenyl, 2-methyl-3-butenyl, 3-methyl-2-butenyl, 3-methyl-3-butenyl, 1-ethyl-2-propenyl and 1-hexenyl. Those alkynyl groups that have 3 to 6 carbon atoms are preferably used as the alkynyl group which may be represented by each of R³ and R⁴. Examples of such alkynyl groups are 2-propynyl, 2-butynyl, 3-butynyl, 2-pentynyl, 3-pentynyl, 4-pentynyl, 1-methyl-3-butynyl, 2-methyl-3-butynyl and 2-hexynyl. These alkenyl groups and alkynyl groups may be substituted. Examples of the substituting groups for this purpose are hydroxy group; halogen atoms, e.g. fluorine, chlorine, bromine and iodine; alkoxy groups, e.g. methoxy, ethoxy, propoxy and butoxy; and aralkyloxy groups, e.g. benzyloxy.

Those aralkyl groups that consist of an alkyl part of alkyl group having 1 to 6 carbon atoms and an aryl part of aryl group having 6 to 10 carbon atoms are preferably used as the aralkyl group which may be represented by each of R³ and R⁴. Examples of such aralkyl groups are benzyl, 1-phenylethyl and naphthylmethyl. These aralkyl groups may be substituted. Examples of the substituting groups for this purpose are hydroxy group; halogen atoms, e.g. fluorine, chlorine, bromine and iodine; alkyl groups, e.g. methyl, ethyl, n-propyl, isopropyl, n-butyl, isobutyl, sec-butyl and tert-butyl; alkoxy groups, e.g. methoxy, ethoxy, propoxy and butoxy; and aralkyloxy groups, e.g. benzyloxy.

Those alkylene groups that have 1 to 6 carbon atoms are preferably used as the alkylene group which may be represented by combination of R³ and R⁴ and may be substituted. Examples of such alkylene groups are methylene, ethylene, methylethylene, 1,2-dimethylethylene, trimethylene, 1-methyltrimethylene, 2-methyltrimethylene, 2,2-dimethyltrimethylene, tetramethylene, pentamethylene and hexamethylene.

The process steps are now each described.
1. The process which comprises reacting an alkali metal or an alkali earth metal with compound (I) or compound (I-1) which is an embodiment of the compound (I), in the presence of ammonia or an amine, to obtain compound (II) or compound (II-1) which is an embodiment of compound (II).

Example of the amine usable for the above purpose are methylamine, dimethylamine, trimethylamine, ethylamine, diethylamine, triethylamine, ethyldimethylamine, diethylmethylamine, isopropylamine, diisopropylamine, triisopropylamine, isopropyldimethylamine, diisopropylethylamine, tributylamine and tripentylamine. There is no particular limitation to the amount of the ammonia or amine, but, generally, the amount is desirably 1 to 200 times by weight based on the weight of compound (I) used.

Examples of the alkali metal usable for the above purpose are lithium, sodium, potassium and rubidium; and those of the alkali earth metals are calcium, strontium and barium. These alkali metals or alkali earth metals may be used desirably in an amount of at least 2 gram atoms based on 1 mole of the compound (I), more preferably in a range of 2 to 20 gram atoms on the same basis.

It is desirable to permit a metal amide or metal hydride of the above alkali metal or alkali earth metal to be present in combination in the reaction zone. Examples of the metal amide are lithium amide, lithium diisopropylamide, sodium amide, sodium diisopropylamide, potassium diisopropylamide, lithium dicyclohexylamide, sodium dicyclohexylamide, potassium dicyclohexylamide, lithium bis(trimethylsilyl)amide, sodium bis(trimethylsilyl)amide and potassium bis(trimethylsilyl)amide. Examples of the metal hydride are lithium hydride, sodium hydride, potassium hydride, magnesium hydride and calcium hydride. These metal amide or metal hydride of the alkali metal or alkali earth metal are, when used in combination therewith, used desirably in an amount of not more than 20 moles based on 1 mole of the compound (I).

The reaction zone may further contain an ammonium salt, the hydroxide of the above alkali metal or alkali earth metal, or a salt of the above alkali metal or alkali earth metal. Examples of the ammonium salt are ammonium chloride, ammonium bromide, ammonium iodide and ammonium carbonate. Examples of the hydroxide of the alkali metal or alkali earth metal are lithium hydroxide, sodium hydroxide, potassium hydroxide and calcium hydroxide. Examples of the salt of the alkali metal or alkali earth metal are metal halides, e.g. lithium chloride, lithium bromide, lithium iodide, sodium chloride, sodium bromide, sodium iodide, potassium chloride, potassium bromide, potassium iodide, calcium chloride, calcium bromide and calcium iodide; metal carbonates, e.g. lithium carbonate, sodium carbonate, potassium carbonate and calcium carbonate; and metal hydrogencarbonates, e.g. lithium hydrogencarbonate, sodium hydrogencarbonate, potassium hydrogencarbonate and calcium hydrogencarbonate.

The reaction can be carried out either in the presence or absence of a solvent. Any solvent can be used for this purpose with no specific restrictions, as long as it does not influence the reaction badly. Examples of usable solvents are alcohols, e.g. methanol, ethanol, n-propanol and isopropanol; ethers, e.g. tetrahydrofuran, diethyl ether and dimethoxyethane; hydrocarbons, e.g. pentane, hexane, heptane, octane, petroleum ether, benzene and toluene; and mixtures of the foregoing. Where an solvent is used, the amount of its use is not particularly limited but, generally, the amount is desirably in a range of 1 to 100 times by weight based on the weight of the compound (I) used.

The reaction temperature is desirably in a range of -100 to 200°C , more preferably in a range of -80 to 30°C.

It is desirable to effect the reaction, for example by adding an alkali metal or an alkali earth metal, under an atmosphere of an inert gas such as nitrogen, or argon, to ammonia or an amine, and further, as necessary, adding a solvent to suspend or dissolve, to prepare a mixture, and adding to the mixture compound (I) or a liquid obtained by suspending or dissolving the compound (I) in an amine or a solvent. Where an ammonium salt, hydroxide or salt of an alkali metal or alkali earth metal, or a metal amide or metal hydride is permitted to be present in combination in the reaction zone, it may be added either at the start of the reaction or during the reaction.

The thus obtained compound (II) or compound (II-1) that is an embodiment of the compound (II) can be isolated and purified according to any one of the usual processes for isolating and purifying organic compounds. For example, the reaction is terminated by adding an alcohol such as methanol or ethanol, or an ammonium salt such as ammonium chloride or ammonium bromide, the reaction mixture is poured into salt water or water, and the obtained mixture is subjected to extraction with an organic solvent such as diethyl ether, ethyl acetate or methylene chloride. The extract is, as necessary, washed with saturated aqueous sodium hydrogencarbonate solution or the like to remove acidic substances and then washed with dilute hydrochloric acid, water or salt water to remove basic substances and water-soluble substances. The washed product is then dried over anhydrous magnesium sulfate, anhydrous sodium sulfate or the like and condensed, to yield a crude product, which is as necessary further purified by distillation, chromatography, recrystallization or like processes.

The thus obtained compound (II), i.e. 7 α ,21-dihydroxy-20-methyl-5 α -pregna-3-one, as well as compound (II-1), i.e. (20S)-7 α ,21-dihydroxy-20-methyl-5 α -pregna-3-one which is an embodiment of the compound (II), is a novel compound.
2. The process which comprises protecting the hydroxy groups of compound (II) or compound (II-1) which is an embodiment of the compound (II), to obtain 3-oxopregnane derivative (III) or 3-oxopregnane derivative (III-1) which is an embodiment of the 3-oxopregnane derivative (III).

The protection of the hydroxy groups, i.e. the 7-position and 22-position hydroxy groups, of compound (II) or compound (II-1) which is an embodiment of the compound (II) can be carried out by any one of processes usually employed for protecting hydroxy group.

For example, where any one of the above-described alkyl groups or aralkyl groups is used for the protection, the reaction is carried out by reacting compound (II) or compound (II-1) which is an embodiment of the compound (II), with an alkyl halide, e.g. tert-butyl chloride and tert-amyl chloride; or an aralkyl halide, e.g. benzyl chloride, benzyl bromide, o-methylbenzyl chloride, m-methylbenzyl chloride, p-methylbenzyl chloride, p-nitrobenzyl chloride, p-methoxybenzyl chloride, p-phenylbenzyl chloride, diphenylmethyl chloride and triphenylmethyl chloride, in the presence of a base, such as a metal hydride, e.g. lithium hydride, sodium hydride, potassium hydride and calcium hydride; an alkali metal, e.g. lithium, sodium and potassium; or an alkali earth metal, e.g. magnesium and calcium. The amount of the base used is desirably at least 1 mole based on 1 mole of the alkyl halide or aralkyl halide used, more preferably in a range of 1 to 10 moles on the same basis.

Where any one of the above acyl groups is used for the protection of the hydroxy groups, the reaction is carried out by reacting compound (II) or compound (II-1) which is an embodiment of the compound (II), with an acid halide, e.g. acetyl chloride, acetyl bromide, chloroacetyl chloride, dichloroacetyl chloride, trichloroacetyl chloride, trifluoroacetyl chloride, methoxyacetyl chloride, triphenylmethoxyacetyl chloride, triphenylmethoxyacetyl bromide, phenoxyacetyl chloride, p-chlorophenoxyacetyl chloride, phenylacetyl chloride, phenylacetyl bromide, diphenylacetyl chloride, propionyl chloride, butyryl chloride, valeryl chloride, 4-pentenoyl chloride, pivaloyl chloride, crotonoyl chloride, benzoyl chloride, benzoyl bromide, o-methylbenzoyl chloride, m-methylbenzoyl chloride, p-methylbenzoyl chloride, 2,3-dimethylbenzoyl chloride, 2,4-dimethylbenzoyl chloride, 2,5-dimethylbenzoyl chloride, 2,6-dimethylbenzoyl chloride, 2,4,6-trimethylbenzoyl chloride and p-phenylbenzoyl chloride; or an acid anhydride, e.g. acetic anhydride, chloroacetic anhydride, dichloroacetic anhydride, trichloroacetic anhydride, trifluoroacetic anhydride, methoxyacetic anhydride, triphenylmethoxyacetic anhydride, phenoxyacetic anhydride, p-chlorophenoxyacetic anhydride, phenylacetic anhydride, diphenylacetic anhydride, 4-pentenic anhydride, pivalic anhydride, crotonic anhydride, benzoic anhydride and p-phenylbenzoic anhydride, in the presence of a base, such as a metal hydride, e.g. lithium hydride, sodium hydride, potassium hydride and calcium hydride; an alkali metal, e.g. lithium, sodium and potassium; an alkali earth metal, e.g. magnesium and calcium; or an amine, e.g. trimethylamine, triethylamine, diisopropylethylamine, pyridine, N,N-dimethylaminopyridine, N,N-dimethylaniline and N,N-diethylaniline. The amount of the base used is desirably at least 1 mole based on 1 mole of the acid halide or acid anhydride used, more preferably in a range of 1 to 10 moles on the same basis.

Where any one of the above alkoxycarboxyl groups, alkenyloxycarbonyl groups, aryloxycarbonyl groups and aralkyloxycarbonyl groups is used for the protection of the hydroxy groups, the reaction is carried out by reacting compound (II) or compound (II-1) which is an embodiment of the compound (II), with an alkoxycarbonyl halide, e.g. methyl chloroformate, ethyl chloroformate, 2,2,2-trichloroethyl chloroformate, 9-fluorenylmethyl chloroformate, 1,1-dimethyl-2,2,2-trichloroethyl chloroformate and 2-(trimethylsilyl)ethyl chloroformate; an alkenyloxycarbonyl halide, e.g. vinyl chloroformate and allyl chloroformate; an aryloxycarbonyl halide, e.g. phenyl chloroformate and p-nitrophenyl chloroformate; or an aralkyloxycarbonyl halide, e.g. benzyl chloroformate, p-methoxybenzyl chloroformate, 3,4-dimethoxybenzyl chloroformate, o-nitrobenzyl chloroformate, p-nitrobenzyl chloroformate, 2-(4-nitrophenyl)ethyl chloroformate and 2-(2,4-dinitrophenyl)ethyl chloroformate, in the presence of a base, such as an amine, e.g. trimethylamine, triethylamine, diisopropylethylamine, pyridine, N,N-dimethylaminopyridine, N,N-dimethylaniline and N,N-diethylaniline. The amount of the base used is desirably at least 1 mole based on 1 mole of the alkoxycarbonyl halide, alkenyloxycarbonyl halide, aryloxycarbonyl halide or aralkyloxycarbonyl halide used, more preferably in a range of 1 to 10 moles on the same basis.

Where any one of the above alkoxyalkyl groups is used for the protection of the hydroxy groups, the reaction is carried out by reacting compound (II) or compound (II-1) which is an embodiment of the compound (II), with an alkoxyalkyl halide, e.g. 1-methoxymethyl chloride, 1-methoxymethyl bromide, benzyloxymethyl chloride, p-methoxybenzyloxymethyl chloride, p-nitrobenzylmethoxymethyl chloride, o-nitrobenzylmethoxymethyl chloride, (4-methoxyphenoxy)methyl chloride, tert-butoxymethyl chloride, 2-methoxyethoxymethyl chloride and 2-(trimethylsilyl)-ethoxymethyl chloride, in the presence of a base, such as a metal hydride, e.g. lithium hydride, sodium hydride, potassium hydride and calcium hydride; an alkali metal, e.g. lithium, sodium and potassium; an alkali earth metal, e.g. magnesium and calcium; or an amine, e.g. trimethylamine, triethylamine, diisopropylethylamine, pyridine, N,N-dimethylaminopyridine, N,N-dimethylaniline and N,N-diethylaniline. The amount of the base used is desirably at least 1 mole based on 1 mole of the alkoxyalkyl halide used, more preferably in a range of 1 to 10 moles on the same basis.

Where any one of the above oxacycloalkyl groups is used for the protection of the hydroxy groups, the reaction is carried out by reacting compound (II) or compound (II-1) which is an embodiment of the compound (II), with an oxacycloalkene, e.g. 2,3-dihydrofuran and 2,3-dihydropyrane, in the presence of an acid. Examples of the acid are sulfonic acids, e.g. methanesulfonic acid, ethanesulfonic acid, propanesulfonic acid, benzenesulfonic acid and p-toluenesulfonic acid; and mineral acids, e.g. hydrochloric acid and sulfuric acid. The amount of the acid used is desirably at least 0.0001 mole based on 1 mole of the oxacycloalkene used, more preferably in a range of 0.001 to 1 mole on the same basis.

Where any one of the above trisubstituted silyl groups is used for the protection of the hydroxy groups, the reaction is carried out by reacting compound (II) or compound (II-1) which is an embodiment of the compound (II), with a trisubstituted silyl halide, e.g. trimethylsilyl chloride, trimethylsilyl bromide, ethyldimethylsilyl chloride, isopropyldimethylsilyl chloride, tert-butyldimethylsilyl chloride, triethylsilyl chloride, tert-butyldiphenylsilyl chloride and triphenylsilyl chloride; or a trisubstituted silyl trifluoromethanesulfonate, e.g. trimethylsilyl trifluoromethanesulfonate, ethyldimethylsilyl trifluoromethanesulfonate, isopropyldimethylsilyl trifluoromethanesulfonate, tert-butyldimethylsilyl trifluoromethanesulfonate, triethylsilyl trifluoromethanesulfonate, tert-butyldiphenylsilyl trifluoromethanesulfonate and triphenylsilyl trifluoromethanesulfonate in the presence of a base, such as a metal hydride, e.g. sodium hydride and potassium hydride; or an amine, e.g. triethylamine, N,N-dimethylaminopyridine, N,N-diethylaminopyridine, imidazole and 2,6-lutidine. The amount of the base used is desirably at least 1 mole based on 1 mole of the trisubstituted silyl halide or trisubstituted silyl trifluoromethanesulfonate used, more preferably in a range of 1 to 10 moles on the same basis.

The reaction for protecting the hydroxy groups of the compound (II) or the compound (II-1) which is an embodiment of the compound (II) can be carried out either in the presence or absence of a solvent. Any solvent can be used for this purpose with no specific restrictions, as long as it influences the reaction badly. Examples of usable solvents are halogenated hydrocarbons, e.g. dichloromethane, dichloroethane, chlorobenzene and dichlorobenzene; ethers, e.g. tetrahydrofuran, diethyl ether and dimethoxyethane; hydrocarbons, e.g. pentane, hexane, heptane, octane, petroleum ether, benzene and toluene; and mixtures of the foregoing. Where an solvent is used, the amount of its use is not particularly limited but, generally, desirably in a range of 1 to 100 times by weight based on the weight of the compound (II) or compound (II-1) which is an embodiment of the compound (II), used.

The reaction temperature is desirably in a range of -80 to 200°C, more preferably in a range of -20 to 180°C.

The 7-position hydroxy group and 22-position hydroxy group of the compound (II) or compound (II-1) which is an embodiment of the compound (II) may be protected either with the same protecting group for hydroxy group or each with a different protecting group for hydroxy group.

The thus obtained 3-oxopregnane derivative (III) or 3-oxopregnane derivative (III-1) which is an embodiment of the 3-oxopregnane derivative (III) can be isolated and purified according to any one of the usual processes for isolating and purifying organic compounds. For example, the reaction mixture is poured into salt water or water, and the obtained mixture is subjected to extraction with an organic solvent such as diethyl ether, ethyl acetate or methylene chloride. The extract is, as necessary, washed with saturated aqueous sodium hydrogencarbonate solution or the like to remove acidic substances and then washed with dilute hydrochloric acid, water or salt water to remove basic substances and water-soluble substances. The washed product is then dried over anhydrous magnesium sulfate, anhydrous sodium sulfate or the like and condensed, to yield a crude product, which is as necessary further purified by distillation, chromatography, recrystallization or like processes.
3. The process which comprises protecting the 3-position carbonyl group of 3-oxopregnane derivative (III) or 3-oxopregnane derivative (III-1) which is an embodiment of the 3-oxopregnane derivative (III), to obtain pregnane derivative (IV) or pregnane derivative (IV-1) which is an embodiment of the pregnane derivative (IV).

The reaction for the protection of the 3-position carbonyl group of 3-oxopregnane derivative (III) or 3-oxopregnane derivative (III-1) which is an embodiment of the 3-oxopregnane derivative (III), to obtain pregnane derivative (IV) or pregnane derivative (IV-1) which is an embodiment of the pregnane derivative (IV), can be carried out by any one of processes usually employed for protecting carbonyl group. For example, there can be employed a process which comprises reacting 3-oxopregnane derivative (III) or 3-oxopregnane derivative (III-1) which is an embodiment of the 3-oxopregnane derivative (III), with an alcohol in the presence of an acid.

Examples of the alcohol are monohydric alcohols, e.g. methanol, ethanol, n-propanol, isopropanol, n-butanol, 2-butanol, 2-methyl-1-propanol, n-pentanol, 2-pentanol, 3-methyl-2-butanol, 2,2-dimethyl-1-propanol, 3-pentanol, n-hexanol, allyl alcohol, 2-buten-1-ol, 3-buten-1-ol, 3-buten-2-ol, 2-penten-1-ol, 3-penten-1-ol, 4-penten-1-ol, 3-penten-2-ol, 4-penten-2-ol, 2-methyl-3-buten-1-ol, 3-methyl-3-buten-1-ol, 3-methyl-3-buten-2-ol, propargyl alcohol, 2-butyn-1-ol, 3-butyn-1-ol, 3-butyn-2-ol, 2-pentyn-1-ol, 3-pentyn-1-ol, 3-pentyn-2-ol, 4-pentyn-1-ol, 2-methyl-3-butyn-1-ol, benzyl alcohol, o-methylbenzyl alcohol, m-methylbenzyl alcohol, p-methylbenzyl alcohol, (2,3-dimethylphenyl)methanol, (2,4-dimethylphenyl)methanol, (2,5-dimethylphenyl)methanol, (2,6-dimethylphenyl)methanol, (2,4,6-trimethylphenyl)methanol, 1-phenylethanol and naphthylmethanol; and polyhydric alcohols, e.g. ethylene glycol, 1,2-propanediol, 1,3-propanediol, 2,2-dimethyl-1,3-propanediol, 1,2-butanediol, 1,3-butanediol, 1,4-butanediol, 1,5-pentanediol and 1,6-hexanediol. The amount of the alcohol used is not particularly limited but, generally, desirably at least 1 mole based on 1 mole of the 3-oxopregnane derivative (III) or 3-oxopregnane derivative (III-1) which is an embodiment of the 3-oxopregnane derivative (III), used. Where a monohydric alcohol is used as the alcohol, its amount is more preferably in a range of 2 to 20 moles; and where a polyhydric alcohol is used, its amount is more preferably in a range of 1 to 20 moles both on the above basis.

Examples of the acid are sulfonic acids, e.g. methanesulfonic acid, ethanesulfonic acid, propanesulfonic acid, benzenesulfonic acid and p-toluenesulfonic acid; and mineral acids, e.g. hydrochloric acid and sulfuric acid. These acids may be used in any amount with no particular restrictions, but, generally, the amount used is desirably in a range of 0.0001 to 1 mole based on 1 mole of the 3-oxopregnane derivative (III) or the 3-oxopregnane derivative (III-1), which is an embodiment of the 3-oxopregnane derivative (III), used, more preferably in a range of 0.001 to 0.5 mole on the same basis.

The reaction can be carried out either in the presence or absence of a solvent. Any solvent can be used for this purpose with no specific restrictions, as long as it does not influence the reaction badly. Examples of usable solvents are ethers, e.g. tetrahydrofuran, diethyl ether and dimethoxyethane; hydrocarbons, e.g. pentane, hexane, heptane, octane, petroleum ether, benzene and toluene; halogenated hydrocarbons, e.g. dichloromethane, chloroform, carbon tetrachloride, dichloroethane, tetrachloroethane, chlorobenzene and o-dichlorobenzene; and mixtures of the foregoing. Where a solvent is used, the amount of its use is not particularly limited but, generally, desirably in a range of 1 to 200 times by weight based on the weight of the 3-oxopregnane derivative (III) or the 3-oxopregnane derivative (III-1) which is an embodiment of the 3-oxopregnane derivative (III), used.

The reaction temperature is desirably in a range of -100 to 200°C, more preferably in a range of -30 to 180°C.

The reaction is desirably effected by mixing an acid, 3-oxopregnane derivative (III) or 3-oxopregnane derivative (III-1) which is an embodiment of the 3-oxopregnane derivative (III), an alcohol and, as necessary, a solvent, and then stirring the mixture at a prescribed temperature.

The thus obtained pregnane derivative (IV) or pregnane derivative (IV-1) which is an embodiment of the pregnane derivative (IV) can be isolated and purified according to any one of the usual processes for isolating and purifying organic compounds. For example, the reaction mixture is poured into salt water or water, and the obtained mixture is subjected to extraction with an organic solvent such as diethyl ether, ethyl acetate or methylene chloride. The extract is, as necessary, washed with saturated aqueous sodium hydrogencarbonate solution or the like to remove acidic substances and then washed with dilute hydrochloric acid, water or salt water to remove basic substances and water-soluble substances. The washed product is then dried over anhydrous magnesium sulfate, anhydrous sodium sulfate or the like and condensed, to yield a crude product, which is as necessary further purified by distillation, chromatography, recrystallization or like processes.
4. The process which comprises subjecting pregnane derivative (IV) or pregnane derivative (IV-1) which is an embodiment of the pregnane derivative (IV) to solvolysis, to obtain 21-hydroxypregnane derivative (V) or 21-hydroxypregnane derivative (V-1) which is an embodiment of the 21-hydroxypregnane derivative (V).

The solvolysis of pregnane derivative (IV) or pregnane derivative (IV-1) which is an embodiment of the pregnane derivative (IV) is desirably carried out in the presence of an alcohol as exemplified in the above process 3, or of water. Methanol, ethanol or isopropanol are particularly preferred as the alcohol used. The alcohol or water can be used in any amount with no particular limitation but, generally, the amount used is preferably in a range of 1 to 200 times by weight based on the weight of the pregnane derivative (IV) or the pregnane derivative (IV-1) which is an embodiment of the pregnane derivative (IV), used.

On effecting the reaction, it is particularly desirable to permit a base or acid to be present in combination in the reaction zone. Examples of the base are metal hydroxides, e.g. sodium hydroxide, potassium hydroxide, lithium hydroxide and barium hydroxide; metal alkoxides, e.g. lithium tert-butoxide, sodium tert-butoxide and potassium tert-butoxide; metal carbonates, e.g. lithium carbonate, sodium carbonate, potassium carbonate and calcium carbonate; and metal hydrogencarbonates, e.g. lithium hydrogencarbonate, sodium hydrogencarbonate, potassium hydrogencarbonate and calcium hydrogencarbonate. Examples of the acid are sulfonic acids, e.g. methanesulfonic acid, ethanesulfonic acid, propanesulfonic acid, benzenesulfonic acid and p-toluenesulfonic acid; mineral acids, e.g. hydrochloric acid and sulfuric acid; Lewis acids, e.g. boron trifluoride, boron trifluoride-diethyl ether complex, aluminum trichloride, titanium trichloride, titanium tetrachloride and zinc bromide; and trifluoroacetic acid. Where a base is used in combination, the amount of its use is not particularly limited but, generally, desirably at least 1 mole based on 1 mole of the pregnane derivative (IV) or the pregnane derivative (IV-1) which is an embodiment of the pregnane derivative (IV), used, more preferably in a range of 1 to 10 moles on the same basis. Where an acid is used in combination, the amount of its use is not particularly limited but, generally, desirably at least 0.001 mole based on 1 mole of the pregnane derivative (IV) or the pregnane derivative (IV-1) which is an embodiment of the pregnane derivative (IV), used, more preferably in a range of 0.01 to 1 mole on the same basis.

The reaction can be carried out either in the presence or absence of a solvent. Any solvent can be used for this purpose with no specific restrictions, as long as it does not influence the reaction badly. Examples of usable solvents are ethers, e.g. tetrahydrofuran, diethyl ether and dimethoxyethane; hydrocarbons, e.g. toluene, pentane, hexane, heptane, octane and petroleum ether; halogenated hydrocarbons, e.g. dichloromethane, chloroform, carbon tetrachloride, dichloroethane, tetrachloroethane, chlorobenzene and o-dichlorobenzene; and mixtures of the foregoing. Where a solvent is used, the amount of its use is not particularly limited but, generally, the amount is desirably in a range of 1 to 200 times by weight based on the weight of the pregnane derivative (IV) or the pregnane derivative (IV-1), which is an embodiment of the pregnane derivative (IV), used.

The reaction temperature is desirably in a range of -100 to 200°C, more preferably in a range of -30 to 180°C.

The reaction is desirably effected by mixing pregnane derivative (IV) or pregnane derivative (IV-1) which is an embodiment of the pregnane derivative (IV), an alcohol or water and, as necessary, a base or an acid and a solvent, and then stirring the mixture at a prescribed temperature.

The thus obtained 21-hydroxypregnane derivative (V) or 21-hydroxypregnane derivative (V-1) which is an embodiment of the 21-hydroxypregnane derivative (V) can be isolated and purified according to any one of the usual processes for isolating and purifying organic compounds. For example, the reaction mixture is poured into salt water or water, and the obtained mixture is subjected to extraction with an organic solvent such as diethyl ether, ethyl acetate or methylene chloride. The extract is, as necessary, washed with saturated aqueous sodium hydrogencarbonate solution or the like to remove acidic substances and then washed with dilute hydrochloric acid, water or salt water to remove basic substances and water-soluble substances. The washed product is then dried over anhydrous magnesium sulfate, anhydrous sodium sulfate or the like and condensed, to yield a crude product, which is as necessary further purified by distillation, chromatography, recrystallization or like processes.
5. The process which comprises subjecting 3-oxopregnane derivative (III) or 3-oxopregnane derivative (III-1) which is an embodiment of the pregnane derivative (III) to solvolysis, to obtain 21-hydroxy-3-oxopregnane derivative (VI) or 21-hydroxy-3-oxopregnane derivative (VI-1) which is an embodiment of the 21-hydroxy-3-oxopregnane derivative (VI).

The solvolysis of 3-oxopregnane derivative (III) or 3-oxopregnane derivative (III-1) which is an embodiment of the 3-oxopregnane derivative (III) is desirably carried out in the presence of an alcohol as exemplified in the above process 3, or of water. Methanol, ethanol or isopropanol is particularly preferred as the alcohol used. The alcohol or water can be used in any amount with no particular limitation but, generally, the amount used is preferably in a range of 1 to 200 times by weight based on the weight of the 3-oxopregnane derivative (III) or the 3-oxopregnane derivative (III-1) which is an embodiment of the 3-oxopregnane derivative (III).

On effecting the reaction, it is particularly desirable to permit a base or an acid to be present in combination in the reaction zone. Examples of the base are metal hydroxides, e.g. sodium hydroxide, potassium hydroxide, lithium hydroxide and barium hydroxide; metal alkoxides, e.g. lithium tert-butoxide, sodium tert-butoxide and potassium tert-butoxide; metal carbonates, e.g. lithium carbonate, sodium carbonate, potassium carbonate and calcium carbonate; and metal hydrogencarbonates, e.g. lithium hydrogencarbonate, sodium hydrogencarbonate, potassium hydrogencarbonate and calcium hydrogencarbonate. Examples of the acid are sulfonic acids, e.g. methanesulfonic acid, ethanesulfonic acid, propanesulfonic acid, benzenesulfonic acid and p-toluenesulfonic acid; mineral acids, e.g. hydrochloric acid and sulfuric acid; Lewis acids, e.g. boron trifluoride, boron trifluoride-diethyl ether complex, aluminum trichloride, titanium trichloride, titanium tetrachloride and zinc bromide; and trifluoroacetic acid. Where a base is used in combination, the amount of its use is not particularly limited but, generally, the amount is desirably at least 1 mole based on 1 mole of the 3-oxopregnane derivative (III) or the 3-oxopregnane derivative (III-1) which is an embodiment of the pregnane derivative (III), used, more preferably in a range of 1 to 10 moles on the same basis. Where an acid is used in combination, the amount of its use is not particularly limited but, generally, the amount is desirably at least 0.001 mole based on 1 mole of the 3-oxopregnane derivative (III) or the 3-oxopregnane derivative (III-1) which is an embodiment of the 3-oxopregnane derivative (III), used, more preferably in a range of 0.01 to 1 mole on the same basis.

The reaction can be carried out either in the presence or absence of a solvent. Any solvent can be used for this purpose with no specific restrictions, as long as it does not influence the reaction badly. Examples of usable solvents are ethers, e.g. tetrahydrofuran, diethyl ether and dimethoxyethane; hydrocarbons, e.g. toluene, pentane, hexane, heptane, octane and petroleum ether; halogenated hydrocarbons, e.g. dichloromethane, chloroform, carbon tetrachloride, dichloroethane, tetrachloroethane, chlorobenzene and o-dichlorobenzene; and mixtures of the foregoing. Where a solvent is used, the amount of its use is not particularly limited but, generally, the amount is desirably in a range of 1 to 200 times by weight based on the weight of the 3-oxopregnane derivative (III) or the 3-oxopregnane derivative (III-1), which is an embodiment of the 3-oxopregnane derivative (III), used.

The reaction temperature is desirably in a range of -100 to 200°C, more preferably in a range of -30 to 180°C.

The reaction is desirably effected by mixing 3-oxopregnane derivative (III) or 3-oxopregnane derivative (III-1) which is an embodiment of the 3-oxopregnane derivative (III), an alcohol or water and, as necessary, a base or an acid and a solvent, and then stirring the mixture at a prescribed temperature.

The thus obtained 21-hydroxy-3-oxopregnane derivative (VI) or 21-hydroxy-3-oxopregnane derivative (VI-1) which is an embodiment of the 21-hydroxy-3-oxopregnane derivative (VI) can be isolated and purified according to any one of the usual processes for isolating and purifying organic compounds. For example, the reaction mixture is poured into salt water or water, and the obtained mixture is subjected to extraction with an organic solvent such as diethyl ether, ethyl acetate or methylene chloride. The extract is, as necessary, washed with saturated aqueous sodium hydrogencarbonate solution or the like to remove acidic substances and then washed with dilute hydrochloric acid, water or salt water to remove basic substances and water-soluble substances. The washed product is then dried over anhydrous magnesium sulfate, anhydrous sodium sulfate or the like and condensed, to yield a crude product, which is as necessary further purified by distillation, chromatography, recrystallization or like processes.
6. The process which comprises protecting the 3-position carbonyl group of 21-hydroxy-3-oxopregnane derivative (VI) or 21-hydroxy-3-oxopregnane derivative (VI-1) which is an embodiment of the 21-hydroxy-3-oxopregnane derivative (VI), to obtain 21-hydroxypregnane derivative (V) or 21-hydroxypregnane derivative (V-1) which is an embodiment of the 21-hydroxypregnane derivative (V).

The protection of the 3-position carbonyl group of 21-hydroxy-3-oxopregnane derivative (VI) or 21-hydroxy-3-oxopregnane derivative (VI-1) which is an embodiment of the 21-hydroxy-3-oxopregnane derivative (VI) can be carried out by any one of processes usually employed for protecting carbonyl group. For example, there can be employed a process which comprises reacting 21-hydroxy-3-oxopregnane derivative (VI) or 21-hydroxy-3-oxopregnane derivative (VI-1) which is an embodiment of the 21-hydroxy-3-oxopregnane derivative (VI), with an alcohol in the presence of an acid.

Examples of the alcohol are monohydric alcohols, e.g. methanol, ethanol, n-propanol, isopropanol, n-butanol, 2-butanol, 2-methyl-1-propanol, n-pentanol, 2-pentanol, 3-methyl-2-butanol, 2,2-dimethyl-1-propanol, 3-pentanol, n-hexanol, allyl alcohol, 2-buten-1-ol, 3-buten-1-ol, 3-buten-2-ol, 2-penten-1-ol, 3-penten-1-ol, 4-penten-1-ol, 3-penten-2-ol, 4-penten-2-ol, 2-methyl-3-buten-1-ol, 3-methyl-3-buten-1-ol, 3-methyl-3-buten-2-ol, propargyl alcohol, 2-butyn-1-ol, 3-butyn-1-ol, 3-butyn-2-ol, 2-pentyn-1-ol, 3-pentyn-1-ol, 3-pentyn-2-ol, 4-pentyn-1-ol, 2-methyl-3-butyn-1-ol, benzyl alcohol, o-methylbenzyl alcohol, m-methylbenzyl alcohol, p-methylbenzyl alcohol, (2,3-dimethylphenyl)methanol, (2,4-dimethylphenyl)methanol, (2,5-dimethylphenyl)methanol, (2,6-dimethylphenyl)methanol, (2,4,6-trimethylphenyl)methanol, 1-phenylethanol and naphthylmethanol; and polyhydric alcohols, e.g. ethylene glycol, 1,2-propanediol, 1,3-propanediol, 2,2-dimethyl-1,3-propanediol, 1,2-butanediol, 1,3-butanediol, 1,4-butanediol, 1,5-pentanediol and 1,6-hexanediol. The amount of the alcohol used is not particularly limited but, generally, desirably at least 1 mole based on 1 mole of the 21-hydroxy-3-oxopregnane derivative (VI) or 21-hydroxy-3-oxopregnane derivative (VI-1) which is an embodiment of the 21-hydroxy-3-oxopregnane derivative (VI), used. Where a monohydric alcohol is used as the alcohol, its amount is more preferably in a range of 2 to 20 moles; and where a polyhydric alcohol is used, its amount is more preferably in a range of 1 to 20 moles, both on the above basis.

Examples of the acid are sulfonic acids, e.g. methanesulfonic acid, ethanesulfonic acid, propanesulfonic acid, benzenesulfonic acid and p-toluenesulfonic acid; and mineral acids, e.g. hydrochloric acid and sulfuric acid. These acids may be used in any amount with no particular restrictions, but, generally, the amount used is desirably in a range of 0.0001 to 1 mole based on 1 mole of the 21-hydroxy-3-oxopregnane derivative (VI) or the 21-hydroxy-3-oxopregnane derivative (VI-1) which is an embodiment of the 21-hydroxy-3-oxopregnane derivative (VI), used, more preferably in a range of 0.001 to 0.5 mole on the same basis.

The reaction can be carried out either in the presence or absence of a solvent. Any solvent can be used for this purpose with no specific restrictions, as long as it does not influence the reaction badly. Examples of usable solvents are ethers, e.g. tetrahydrofuran, diethyl ether and dimethoxyethane; hydrocarbons, e.g. pentane, hexane, heptane, octane, petroleum ether, benzene and toluene; halogenated hydrocarbons, e.g. dichloromethane, chloroform, carbon tetrachloride, dichloroethane, tetrachloroethane, chlorobenzene and o-dichlorobenzene; and mixtures of the foregoing. Where a solvent is used, the amount of its use is not particularly limited but, generally, the amount is desirably in a range of 1 to 200 times by weight based on the weight of the 21-hydroxy-3-oxopregnane derivative (VI) or the 21-hydroxy-3-oxopregnane derivative (VI-1) which is an embodiment of the 21-hydroxy-3-oxopregnane derivative (VI), used.

The reaction temperature is desirably in a range of -100 to 200°C, more preferably in a range of -30 to 180°C .

The reaction is desirably effected by mixing an acid, 21-hydroxy-3-oxopregnane derivative (VI) or 21-hydroxy-3-oxopregnane derivative (VI-1) which is an embodiment of the 21-hydroxy-3-oxopregnane derivative (VI), an alcohol and, as necessary, a solvent, and then stirring the mixture at a prescribed temperature.

The thus obtained 21-hydroxypregnane derivative (V) or 21-hydroxypregnane derivative (V-1) which is an embodiment of the 21-hydroxypregnane derivative (V) can be isolated and purified according to any one of the usual processes for isolating and purifying organic compounds. For example, the reaction mixture is poured into salt water or water, and the obtained mixture is subjected to extraction with an organic solvent such as diethyl ether, ethyl acetate or methylene chloride. The extract is, as necessary, washed with saturated aqueous sodium hydrogencarbonate solution or the like to remove acidic substances and then washed with dilute hydrochloric acid, water or salt water to remove basic substances and water-soluble substances. The washed product is then dried over anhydrous magnesium sulfate, anhydrous sodium sulfate or the like and condensed, to yield a crude product, which is as necessary further purified by distillation, chromatography, recrystallization or like processes.

The 21-hydroxypregnane derivative (V-1), which is an embodiment of 21-hydroxypregnane derivative (V), includes, for example, (20S)-21-hydroxy-3-(spiro-2'-(1',3'-dioxolane))-20-methyl-5 α -pregna- 7 α -ol benzoate. This compound derives, on oxidation of its 22 position, (20S)-20-formyl-3-(spiro-2'-(1',3'-dioxolane))-5 α -pregna-7 *α* -ol benzoate (see Reference Example 2). The derived compound further derives, by reaction with diethylphosphono-3-methyl-2-butanone, (22E)-24-oxo-3-(spiro-2'-(1',3'-dioxolane))-5 *α* -cholest-22-en-7 *α* -ol benzoate, which is further converted, by the processes described in J. Org. Chem. 63, 3786(1998) and J. Org. Chem. 63, 8599(1998), into squalamine (see the scheme below).

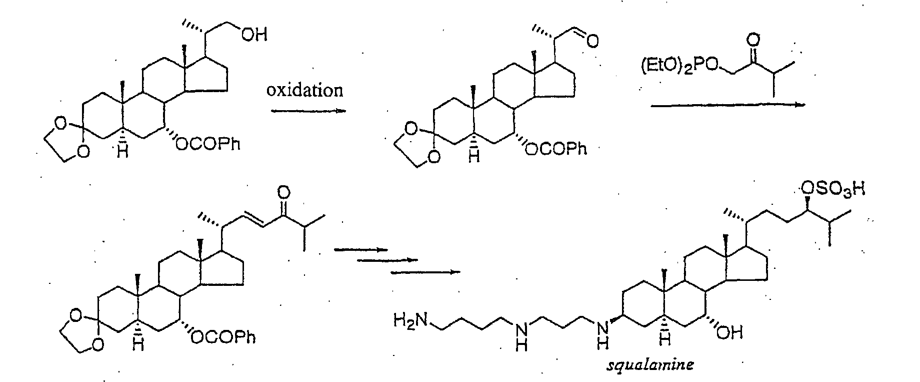

Hereinbelow, the present invention is described with reference to Examples, which are by no means limitative of the invention.

### Reference Example 1

Synthesis of (20S)-7 α ,21-dihydroxy-20-methyl-pregna-1,4-dien-3-one (compound (I))

To 20.0 g (54.8 mmoles) of (20S)-7 α -hydroxy-3-oxo-pregna-1,4-dien-20-carbaldehyde, 200 ml of ethanol was added, and the mixture was ice-cooled with stirring. To the obtained solution, 0.61 g (16.1 mmoles) of sodium borohydride was added in several portions. After completion of the addition, the mixture was stirred for 1 hour under ice-cooling. The resulting reaction mixture was neutralized by addition of 3% hydrochloric acid. After further addition of 200 ml of water, ethanol was distilled off under reduced pressure. The crude crystal that had formed in the residue was recovered by filtration and washed with water. Toluene was added to the obtained crude crystal and the mixture was heated, so that water present in the crude crystal was removed by azeotropy with toluene. Thereafter, toluene was distilled off under reduced pressure, to obtain 18.8 g (yield: 93%) of (20S)-7 α ,21-dihydroxy-20-methyl-pregna-1,4-dien-3-one having the following properties.
¹H-NMR spectrum (270 MHz, CDCl₃, TMS, ppm) δ : 0.765 (s, 3H), 1.048 (d, 3H, J=6.6 Hz), 1.238 (s, 3H), 2.490 (dd, 1H, J=3.3, 13.9 Hz), 2.737 (ddd, 1H, J=1.8, 3.3, 13.9 Hz), 3.366 (dd, 1H, J=6.9, 10.2 Hz), 3.635 (dd, 1H, J=3.3, 10.2 Hz), 4.041 (brs, 1H), 6.143 (m, 1H), 6.253 (dd, 1H, J=1.8, 10.1 Hz), 7.072 (d, 1H, J=10.1 Hz)

### Example 1

Synthesis of (205)-7 α ,21-dihydroxy-20-methyl-5 α -pregna-3-one (compound (II))

A 200-ml three-necked flask was charged with liquid ammonia (70 ml) under an atmosphere of nitrogen and at -78°C and, then, gradually with 0.403 g (58.1 mmoles) of lithium metal while the inside temperature was maintained at -78°C. After the lithium metal had been dissolved completely, to the mixture there was added dropwise gradually a liquid prepared by suspending 2.00 g (5.81 mmoles) of the (20S)-7 α ,21-dihydroxy-20-methyl-pregna-1,4-dien-3-one obtained by the process described in Reference Example 1, in 50 ml of tetrahydrofuran. After completion of the addition, the mixture was stirred for 2 hours at -78°C. To the reaction mixture, 3.73 g (69.7 mmoles) of ammonium chloride was added and, then, the obtained reaction liquid was stirred for 12 hours, while being allowed to be gradually warmed up to room temperature, to remove ammonia. To the obtained white residue 50 ml of toluene and 100 ml of 3% hydrochloric acid were added to dissolve it. After separation of an organic layer from an aqueous layer, the organic layer was washed with saturated salt water and dried over anhydrous magnesium sulfate and then condensed, to give 2.09 g of a crude product. The crude product obtained was purified by silica gel column chromatography (eluent: ethyl acetate /hexane = 2/1 by volume) to yield 1.32 g of (20S)-7 α ,21-dihydroxy-20-methyl-5 α -pregna-3-one having the following properties (yield: 65%).

The thus obtained compound was identified to be a 5 α isomer by the following method. That is, the ¹H-NMR spectrum of the compound was compared with that of (20S)-7 α ,21-dihydroxy-20-methyl-5 β -pregna-3-one, which had been synthesized according to the process described in EP 0 018 515 A2, to reveal that the peak positions based on the 18-position and 19-position methyl groups differed from each other.
(20S)-7 α ,21-dihydroxy-20-methyl-5 α -pregna-3-one
¹H-NMR spectrum (270 MHz, CDCl₃, TMS, ppm) δ : 0.710 (s, 3H), 1.007 (s, 3H), 1.044 (d, 3H, J=6.9 Hz), 1.0-2.5 (m, 22H), 3.339 (dd, 1H, J=6.9, 10.9 Hz), 3.614 (dd, 1H, J=3.0, 10.9 Hz), 3.84-3.85 (brs, 1H).
(20S)-7 α ,21-dihydroxy-20-methyl-5 β -pregna-3-one
¹H-NMR spectrum (270 MHz, CDCl₃, TMS, ppm) δ : 0.729 (s, 3H), 1.014 (s, 3H), 1.070 (d, 3H, J=6.9 Hz), 1.0-2.3 (m, 21H), 2.414 (ddd, 1H, J=5.0, 13.9, 13.9 Hz), 3.30-3.51 (m, 1H), 3.60-3.80 (m, 1H), 3.932 (ddd, 1H, J=3.0, 3.0, 5.9 Hz).

### Example 2

### Synthesis of (20S)-3-oxo-20-methyl-5 α -pregna-7 α ,21-diol bisbenzoate

A 50-ml flask was, under an atmosphere of nitrogen, charged with 294 mg (0.84 mmole) of the (20S)-7 α ,21-dihydroxy-20-methyl-5 α -pregna-3-one obtained by the process described in Example 1, 101 mg (0.84 mmole) of 4-dimethylaminopyridine, 0.953 ml of pyridine and 5 ml of dichloromethane. The mixture was dissolved with stirring and then ice-cooled. To the solution 888 mg (6.32 mmoles) of benzoyl chloride was gradually added dropwise. After completion of the addition, the mixture was stirred for 15 minutes under ice-cooling, then allowed to be warmed up to room temperature, and further stirred for 12 hours. After the reaction, the reaction mixture was poured into 70 ml of saturated aqueous sodium hydrogencarbonate solution, and the resulting organic layer was separated from the aqueous layer. The organic layer was washed with 70 ml of saturated aqueous sodium hydrogencarbonate solution and then with 50 ml of 1N hydrochloric acid. The washed product was dried over anhydrous magnesium sulfate and condensed, to give 420 mg of crude (20S)-3-oxo-20-methyl-5 α -pregna-7 α ,21-diol bisbenzoate having the following properties (crude yield: 90%).
¹H-NMR spectrum (270 MHz, CDCl₃, TMS, ppm) δ : 0.769 (s, 3H), 1.091 (s, 3H), 1.112 (d, 3H, J=6.9 Hz), 1.20-2.50 (m, 22H), 3.983 (dd, 1H, J=7.9, 10.9 Hz), 4.293 (dd, 1H, J=4.0, 10.9 Hz), 5.210 (brd, 1H, J=2.0 Hz), 7.35-7.65 (m, 6H), 8.015 (d, 4H, J=7.9 Hz).

### Example 3

### Synthesis of (20S)-3-(spiro-2'-(1',3'-dioxolane))-20-methyl-5 α -pregna-7α,21-diol bisbenzoate

A 100-ml flask was charged with 1.34 g (2.41 mmoles) of the crude (20S)-3-oxo-20-methyl-5 α -pregna-7 α ,21-diol bisbenzoate obtained by the process given in Example 2 and 50 ml of toluene and the mixture was dissolved. To the solution, 1.76 g (28.4 mmoles) of ethylene glycol and 80 mg (0.42 mmole) of p-toluenesulfonic acid, and the mixture was refluxed for 2 hours. The reaction liquid was washed twice each time with saturated aqueous sodium hydrogencarbonate solution, then dried over anhydrous magnesium sulfate and condensed, to give 1.41 g of crude (20S)-20-methyl-3-(spiro-2'-(1',3'-dioxolane))-5 α -pregna-7 α ,21-diol bisbenzoate having the following properties (crude yield: 97%).
¹H-NMR spectrum (270 MHz, CDCl₃, TMS, ppm) δ : 0.736 (s, 3H), 0.889 (s, 3H), 1.107 (d, 3H, J=5.9 Hz), 1.10-2.10 (m, 22H), 3.80-3.95 (m, 4H), 3.965 (dd, 1H, J=7.9, 10.9 Hz), 4.286 (dd, 1H, J=3.0, 10.9 Hz), 5.174 (brd, 1H; J=2.0 Hz), 7.35-7.61 (m, 6H), 8.013 (d, 2H, J=6.9 Hz), 8.067 (d, 2H, J=6.9 Hz).

### Example 4

### Synthesis of (20S)-21-hydroxy-20-methyl-3-(spiro-2'-(1',3'-dioxolane))-5 α -pregna-7 α -ol benzoate

A 3-1 flask was charged with 32.3 g (53.76 mmoles) of the crude (20S)-20-methyl-3-(spiro-2'-(1',3'-dioxolane))-5 α -pregna-7 α ,21-diol bisbenzoate obtained by the process given in Example 3, 855 ml of methanol and 420 ml of tetrahydrofuran, and the mixture was dissolved. To the solution, a solution of 21.5 g (537 mmoles) of sodium hydroxide in 855 ml of methanol was added dropwise over 4 hours at room temperature. After completion of the addition, the mixture was stirred for 7 hours at room temperature. The reaction mixture was neutralized by addition of 3% hydrochloric acid and then condensed. The residue was extracted with dichloromethane (1 liter x 3). The extracts were combined and dried over anhydrous magnesium sulfate and then condensed, to give a crude product. The crude product obtained was purified by silica gel column chromatography (eluent: ethyl acetate/hexane = 1/4 by volume) to give 22.89 g of (20S)-21-hydroxy-20-methyl-3-(spiro-2'-(1',3'-dioxolane))-5 α -pregna-7 α -ol benzoate having the following properties (yield: 86%).
¹H-NMR spectrum (270 MHz, CDCl₃, TMS, ppm) δ : 0.699 (s, 3H), 0.882 (s, 3H), 1.039 (d, 3H, J=6.9 Hz), 1.00-2.10 (m, 22H), 3.328 (dd, 1H, J=5.9, 9.9 Hz), 3.577 (dd, 1H, J=3.0, 9.9 Hz), 3.83-3.93 (m, 4H), 5.159 (brd, 1H, J=2.0 Hz), 7.48 (dd, 2H, J=6.9, 6.9 Hz), 7.59 (d, 1H, J=6.9 Hz), 8.056 (d, 2H, J=6.9 Hz).

### Example 5

### Synthesis of (20S)-21-hydroxy-20-methyl-3-oxa-5 α -pregna-7 α -ol benzoate

A 300-ml three-necked flask was charged with 34.7 g (62.3 mmoles) of the (20S)-20-methyl-3-oxa-5 α -pregna-7 α ,21-diol bisbenzoate obtained by the process given in Example 2 and then with 130 ml of tetrahydrofuran. To the obtained solution, 11.2 g (81.0 mmoles) of potassium carbonate, 13.6 g (340 mmoles) of sodium hydroxide, 69 ml of water and 69 ml of methanol. The mixture was then stirred for 4 hours at 40°C. After completion of the reaction, the reaction mixture was condensed. To the condensed mixture, 200 ml of water and 300 ml of ethyl acetate were added, and the mixture was stirred. The resulting aqueous layer was separated and then extracted with 200 ml of ethyl acetate. The organic layer was combined with the extract. The layer was then washed twice each time with 100 ml of saturated aqueous sodium hydrogencarbonate solution, then washed with 100 ml of water and dried over anhydrous magnesium sulfate. After filtration, the product was condensed, to give 19.5 g of crude (20S)-21-hydroxy-20-methyl-3-oxa-5 α -pregna-7 *α* -ol benzoate having the following properties (crude yield: 69.5%).
¹H-NMR spectrum (270 MHz, CDCl₃, TMS, ppm) δ : 8.006 (2H, d, J=6.92 Hz), 7.557 (1H, dd, J=6.92, 7.94 Hz), 7.475 (2H, d, J=7.94 Hz), 5.192 (1H, bs), 3.587 (1H, dd, J=1.98, 9.90 Hz), 3.341 (1H, dd, J=5.94, 9.90 Hz), 0.9-2.5 (22H, m), 1.080 (3H, s), 1.044 (3H, d, J=5.94 Hz), 0.729 (3H, s).

### Example 6

### Synthesis of (20S)-21-hydroxy-20-methyl-3-(spiro-2'-(1',3'-dioxolane))-5 α -pregna-7 α -ol benzoate

In 166 ml of toluene, there was dissolved 16.59 g (36.65 mmoles) of the (20S)-21-hydroxy-20-methyl-3-oxa-5 α -pregna-7 α-ol benzoate obtained by the process given in Example 5. To the obtained solution, 16.0 g (258 mmoles) of ethylene glycol and 0.49 g (2.6 mmoles) of p-toluenesulfonic acid monohydrate, and the mixture was then stirred for 40 hours under reflux with heating. After completion of the reaction, the reaction mixture was added to 100 ml of saturated aqueous sodium hydrogencarbonate solution, and washed. After separation of the resulting aqueous layer, the organic layer was washed with 100 ml of saturated salt water. The organic layer was then dried over anhydrous sodium sulfate. After filtration, the product was condensed to give 16.2 g of a crude product. The crude product obtained was purified by silica gel column chromatography (eluent: ethyl acetate/hexane = 1/6) to give 5.16 g of (20S)-21-hydroxy-20-methyl-3-(spiro-2'-(1',3'-dioxolane))-5 α -pregna-7 α -ol benzoate having the following properties (yield: 28%).
¹H-NMR spectrum (270 MHz, CDCl₃, TMS, ppm) δ : 0.699 (s, 3H), 0.882 (s, 3H), 1.039 (d, 3H, J=6.9 Hz), 1.00-2.10 (m, 22H), 3.328 (dd, 1H, J=5.9, 9.9 Hz), 3.577 (dd, 1H, J=3.0, 9.9 Hz), 3.83-3.93 (m, 4H), 5.159 (brd,1H, J=2.0 Hz), 7.48 (dd, 2H, J=6.9, 6.9 Hz), 7.59 (d, 1H, J=6.9 Hz), 8.056 (d, 2H, J=6.9 Hz).

### Reference Example 2

### Synthesis of (20S)-20-formyl-3-(spiro-2'-(1'3'-dioxolane))-5 α-pregna-7 α -ol benzoate

In 214 ml of dichloromethane, there were dissolved 21.4 g (43 mmoles) of the (20S)-21-hydroxy-20-methyl-3-(spiro-2'-(1',3'-dioxolane))-5 α -pregna-7 α -ol benzoate obtained by the process given in Example 4 and 0.13 g (0.8 mmole) of 2,2,6,6-tetramethyl-1-piperidine oxide, and the solution was ice-cooled. To the solution, a solution of 0.51 g (4.3 mmoles) of potassium bromide in 21 ml of water was added under ice-cooling, and the mixture was vigorously stirred. To the mixture, an aqueous solution prepared by adding sodium hydrogencarbonate (1.0 g) to an aqueous sodium hypochlorite solution (2.64% by weight, 108 ml, 40 mmoles) was added dropwise under ice-cooling. After completion of the addition, the mixture was allowed to react for 2 hours under ice-cooling. After completion of the reaction, the resulting aqueous layer was separated from the organic layer. The aqueous layer was extracted with dichloromethane (30 ml x 2). The extracts were combined with the organic layer, and the layer was washed with water (50 ml x 2) and then with saturated salt water (20 ml). The washed product was dried over anhydrous magnesium sulfate and then condensed, to give a crude product. The crude product obtained was purified by silica gel column chromatography (eluent: ethyl acetate/hexane = 1/4 by volume) to give 16.9 g of (20S)-20-formyl-3-(spiro-2'-(1',3'-dioxolane))-5 α -pregna-7 α -ol benzoate having the following properties (yield: 79%).
¹H-NMR spectrum (270 MHz, CDCl₃, TMS, ppm) δ : 0.725 (s, 3H), 0.886 (s, 3H), 1.108 (d, 3H, J=6.9 Hz), 1.13-1.97 (m, 21H), 2.30-2.38 (m, 1H), 3.830-3.929 (m, 4H), 5.166 (m, 1H), 7.483 (dd, 2H, J=6.9, 6.9 Hz), 7.580 (ddd, 1H, J=2.0, 6.9, 6.9 Hz), 8.050 (dd, 2H, J=2.0, 6.9 Hz), 9.518 (d, 1H, J=3.0 Hz).

### INDUSTRIAL APPLICABILITY

According to the present invention, pregnane derivatives useful as synthesis intermediates for squalamine can be prepared from readily available starting materials and efficiently in short steps.

## Claims

1. A process for preparing 21-hydroxypregnane derivatives represented by the general formula (V) 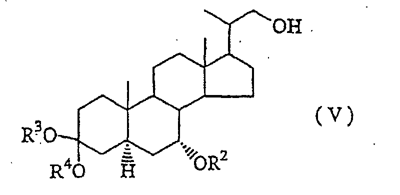 wherein R² represents a protecting group for hydroxy group, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted, which comprises the steps of:
reacting an alkali metal or an alkali earth metal with a compound represented by the formula (I) 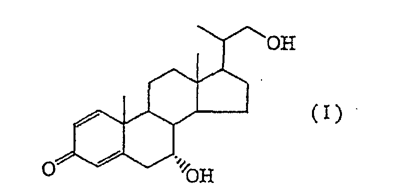
in the presence of ammonia or an amine, to obtain a compound represented by the formula (II); 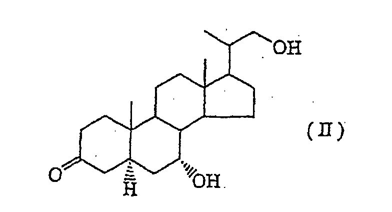
protecting the hydroxy groups of the obtained compound, to obtain a 3-oxopregnane derivative represented by the general formula (III) 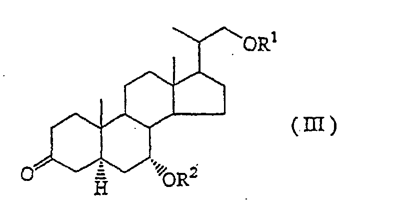
wherein R¹ represents a protecting group for hydroxy group and R² is as defined above;
protecting the 3-position carbonyl group of the obtained 3-oxopregnane derivative, to obtain a pregnane derivative represented by the general formula (IV) 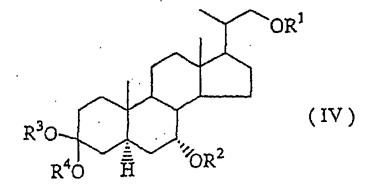 wherein R¹, R², R³ and R⁴ are as defined above; and
subjecting the obtained pregnane derivative to solvolysis.

2. The process according to claim 1, which comprises the steps of:
reacting an alkali metal or an alkali earth metal with a compound represented by the formula (I-1) 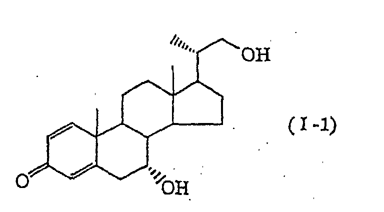
in the presence of ammonia or an amine, to obtain a compound represented by the formula (II-1); 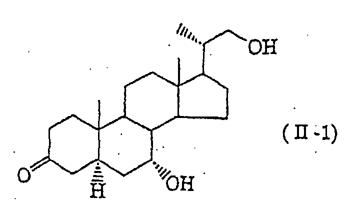
protecting the hydroxy groups of the obtained compound, to obtain a 3-oxopregnane derivative represented by the general formula (III-1) 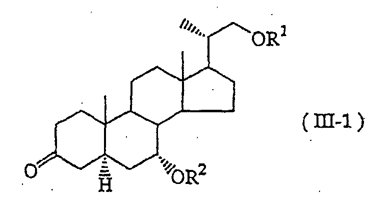
wherein R¹ and R² each represents a protecting group for hydroxy group;
protecting the 3-position carbonyl group of the obtained 3-oxopregnane derivative, to obtain a pregnane derivative represented by the general formula (IV-1) 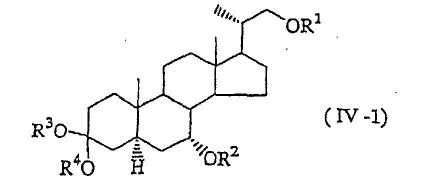 wherein R¹ and R² are as defined above and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted; and
subjecting the obtained pregnane derivative (IV-1) to solvolysis, to obtain a 21-hydroxypregnane derivative represented by the general formula (V-1) 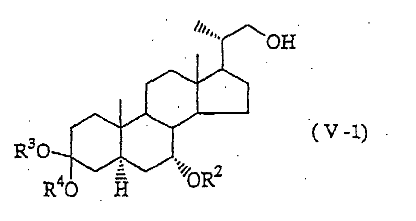 wherein R², R³ and R⁴ are as defined above.

3. A process for preparing compounds represented by the formula (II), 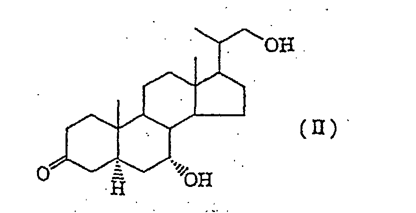 which comprises reacting an alkall metal or an alkali earth metal with a compound represented by the formula (I), 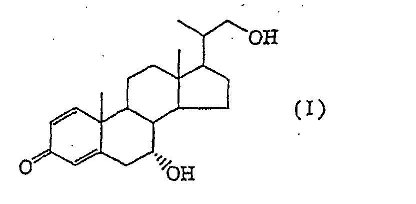 in the presence of ammonia or an amine.

4. The process according to claim 3, which comprises reacting an alkali metal or an alkali earth metal with a compound represented by the formula (I-1), 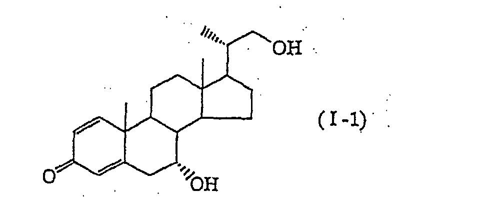 in the presence of ammonia or an amine, to obtain a compounc represented by the formula (II-1).

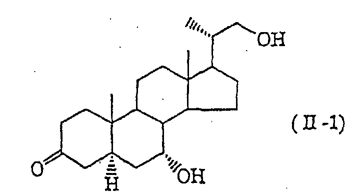

5. A process for preparing pregnane derivatives represented by the general formula (IV) 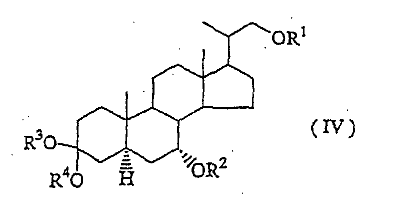 wherein R¹ and R² each represents a protecting group for hydroxy group, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted, which comprises protecting the hydroxy groups of a compound represented by the formula (II), 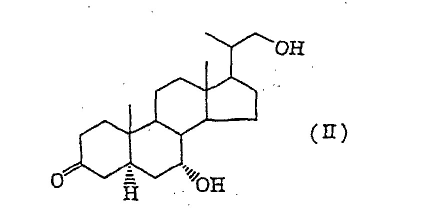 to obtain a 3-oxopregnane derivative represented by the general formula (III) 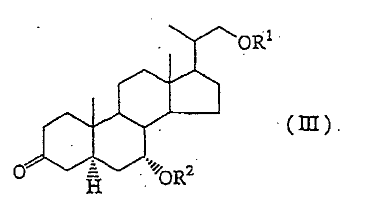 wherein R¹ and R² are as defined above, and protecting the 3-position carbonyl group of the obtained 3-oxopregnane derivative.

6. The process according to claim 5, which comprises protecting the hydroxy groups of a compound represented by the formula (II-1), 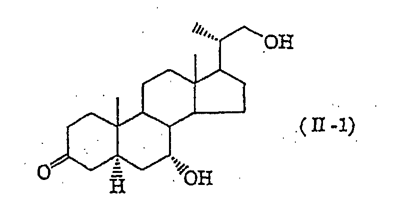 to obtain a 3-oxopregnane derivative represented by the general formula (III-1) 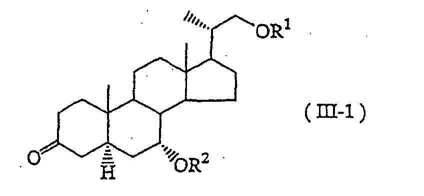 wherein R¹ and R² each represents a protecting group for hydroxy group, and
protecting the 3-position carbonyl group of the obtained 3-oxopregnane derivative (III-1), to obtain a pregnane derivative represented by the general formula (IV-1) 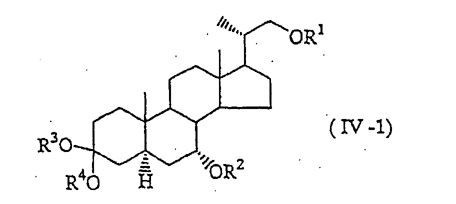 wherein R¹ and R² are as defined above, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted.

7. A process for preparing 21-hydroxypregnane derivatives represented by the general formula (V) 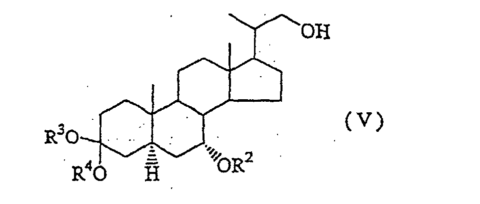 wherein R² represents a protecting group for hydroxy group, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted,
which comprises subjecting a pregnane derivative represented by the general formula (IV) 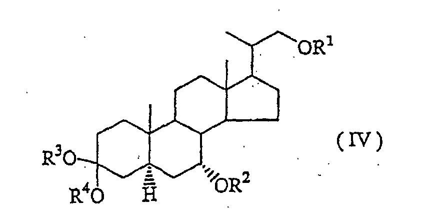 wherein R¹ represents a protecting group for hydroxy group, and R² ,R³ and R⁴ are as defined above, to solvolysis.

8. The process according to claim 7, which comprises subjecting a pregnane derivative represented by the general formula (IV-1) 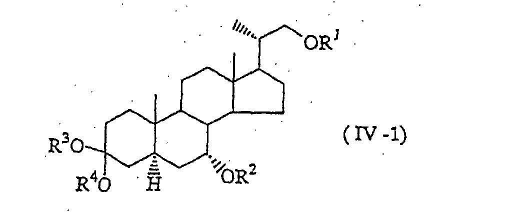 wherein R¹ and R² each represents a protecting group for hydroxy group, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted,
to solvolysis, to obtain a 21-hydroxypregnane derivative represented by the general formula (V-1) 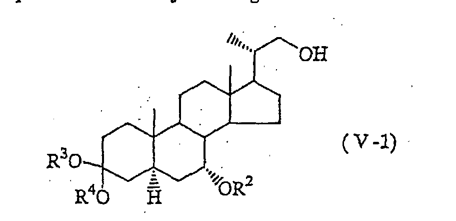 wherein R², R³ and R⁴ are as defined above.

9. 7 α ,21-dihydroxy-20-methyl-5 α -pregna-3-one represented by the formula (II).

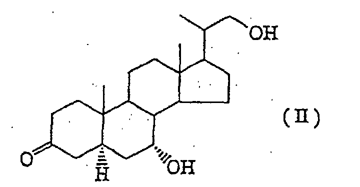

10. The compound according to claim 9, being (20S)-7 α ,21-dihydroxy-20-methyl-5 α -pregna-3-one represented by the formula (II-1).

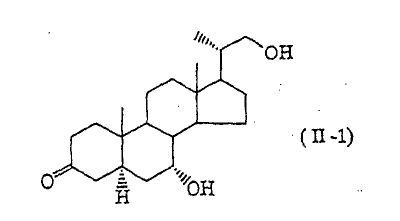

11. A process for preparing 21-hydroxypregnane derivatives represented by the general formula (V) 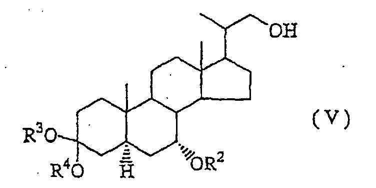 wherein R² represents a protecting group for hydroxy group, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted, which comprises subjecting a 3-oxopregnane derivative represented by the general formula (III) 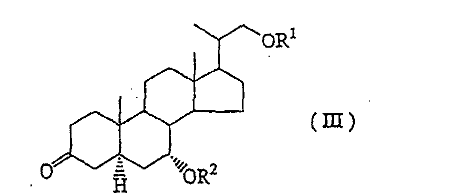 wherein R¹ represents a protecting group for hydroxy group and R² is as defined above, to solvolysis, to obtain a 21-hydroxy-3-oxopregnane derivative represented by the general formula (VI) 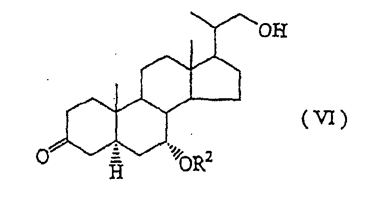 wherein R² is as defined above, and protecting the 3-position carbonyl group of the obtained 21-hydroxy-3-oxopregnane derivative.

12. The process according to claim 11, which comprises subjecting a 3-oxopregnane derivative represented by the general formula (III-1) 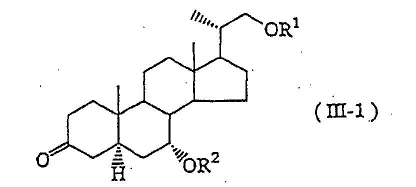 wherein R¹ and R² each represents a protecting group for hydroxy group, to solvolysis, to obtain a 21-hydroxy-3-oxopregnane derivative represented by the general formula (VI-1) 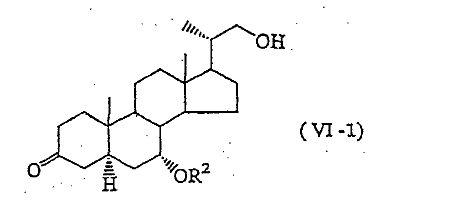 wherein R² is as defined above,
and protecting the 3-position carbonyl group of the obtained 21-hydroxy-3-oxopregnane derivative, to obtain a 21-hydroxypregnane derivative represented by the general formula (V-1) 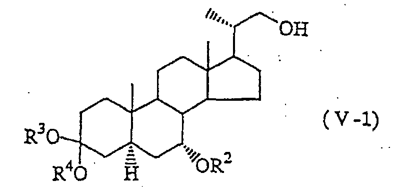 wherein R² is as defined above, and R³ and R⁴ each independently represents an alkyl group which may be substituted, an alkenyl group which may be substituted, an alkynyl group which may be substituted, or an aralkyl group which may be substituted, or R³ and R⁴ jointly represent an alkylene group which may be substituted.
